# EUROPEAN PATENT APPLICATION

(11) **EP 4 717 175 A1**
(43) Date of publication of application: **01.04.2026**
(21) Application number: 24811161.9
(22) Date of filing: 23.05.2024
(51) Int. Cl.: A61B 5/346, G16H 10/40, G16H 50/30

(54) **COMPUTER PROGRAM, INTRACARDIAC ELECTROCARDIOGRAM EVALUATION DEVICE, INTRACARDIAC ELECTROCARDIOGRAM EVALUATION SYSTEM, INTRACARDIAC ELECTROCARDIOGRAM EVALUATION METHOD, AND LEARNING MODEL GENERATION METHOD**

(30) Priority: 23.05.2023 JP 2023084755
(71) Applicant: Kyoto University, Kyoto-shi, Kyoto 606-8501 (JP)
(72) Inventor: KOHJITANI, Hirohiko, Kyoto-shi, Kyoto 606-8501 (JP); TANAKA, Munekazu, Kyoto-shi, Kyoto 606-8501 (JP); SHIZUTA, Satoshi, Kyoto-shi, Kyoto 606-8501 (JP); OKUNO, Yasushi, Kyoto-shi, Kyoto 606-8501 (JP)
(74) Representative: Cabinet Beaumont
(86) International application number: PCT/JP2024/018932
(87) International publication number: WO 2024/242155

(57) **Abstract**

There are provided a computer program, an intracardiac electrocardiogram evaluation apparatus, an intracardiac electrocardiogram evaluation system, an intracardiac electrocardiogram evaluation method, and a learning model generation method capable of predicting with high accuracy the presence or absence of an arrhythmic disease or its mechanism from a body surface electrocardiogram.

The computer program causes a computer to execute processing of: acquiring body surface electrocardiogram data of a subject; and inputting the acquired body surface electrocardiogram data to a first learning model, which outputs an index for evaluating intracardiac electrocardiogram data when body surface electrocardiogram data is input, and outputting an index for evaluating intracardiac electrocardiogram data of the subject.

## Description

### Technical Field

The present invention relates to a computer program, an intracardiac electrocardiogram evaluation apparatus, an intracardiac electrocardiogram evaluation system, an intracardiac electrocardiogram evaluation method, and a learning model generation method.

### Background Art

Atrial fibrillation is an arrhythmic disease that can lead to socially significant and irreversible diseases such as cerebral infarction and heart failure, and is highly prevalent among the elderly. Therefore, in order to reduce overall medical costs and maintain individual productivity toward realizing a healthy and long-lived society, the need for arrhythmia diagnosis is increasing.

Such arrhythmic diseases are usually diagnosed using body surface electrocardiogram examination apparatuses, such as a 12-lead electrocardiograph. Patent Literature 1 discloses an electrocardiogram data processing apparatus that automatically detects a part of a 12-lead electrocardiogram with high accuracy.

### Citation List

### Patent Literature:

Patent Literature 1: Japanese Patent Application Laid-Open No. 2011-72542 Summary

### Technical Problems

However, the body surface electrocardiogram mainly reflects information at the moment of recording and does not necessarily reflect a predisposition to arrhythmia. In other words, since the body surface electrocardiogram cannot provide information at times other than the time of recording, it is difficult to predict the onset of arrhythmic diseases such as atrial fibrillation even if electrocardiographic findings during non-attack periods are obtained.

In addition, although the body surface electrocardiogram is non-invasive and widely used, it is not possible to capture fine electrical information within the heart. On the other hand, an intracardiac electrocardiogram directly records electrical signals obtained from electrode catheters inserted into the heart, allowing detailed representation of structural information about the heart and providing high diagnostic capability. However, since the insertion of a catheter into the body and cardiac chambers is required, an expensive and highly invasive procedure is required.

The present invention has been made in view of the above circumstances, and its object is to provide a computer program, an intracardiac electrocardiogram evaluation apparatus, an intracardiac electrocardiogram evaluation system, an intracardiac electrocardiogram evaluation method, and a learning model generation method capable of predicting with high accuracy the presence or absence of an arrhythmic disease or its mechanism from a body surface electrocardiogram.

### Solution to Problems

The present application includes a plurality of means for solving the above-mentioned problems. As one example, a computer program causes a computer to execute processing of: acquiring body surface electrocardiogram data of a subject; and inputting the acquired body surface electrocardiogram data to a first learning model, which outputs an index for evaluating intracardiac electrocardiogram data when body surface electrocardiogram data is input, and outputting an index for evaluating intracardiac electrocardiogram data of the subject.

### Advantageous Effects of Invention

According to the present invention, the presence or absence of an arrhythmic disease or its mechanism can be predicted with high accuracy from a body surface electrocardiogram.

### Brief Description of Drawings

FIG. 1 is a diagram showing an example of the configuration of an intracardiac electrocardiogram evaluation system.
FIG. 2 is a diagram showing an example of body surface electrocardiogram data.
FIG. 3 is a diagram showing an example of the configuration of a first learning model.
FIG. 4 is a diagram showing an example of intracardiac electrocardiogram data.
FIG. 5 is a diagram showing an example of creating training data for generating a first learning model.
FIG. 6 is a diagram showing an example of a method for generating a first learning model.
FIG. 7 is a diagram showing a first example of a label.
FIG. 8 is a diagram showing a second example of a label.
FIG. 9 shows an example of a pattern of coronary sinus potentials.
FIG. 10 is a diagram showing a third example of a label.
FIG. 11 is a diagram showing an example of an atrial tachycardia pattern.
FIG. 12 is a diagram showing an example of an atrioventricular reentrant tachycardia pattern.
FIG. 13 is a diagram showing an example of visualization of prediction grounds using Grad-CAM.
FIG. 14 is a diagram showing an example of the configuration of a second learning model.
FIG. 15 is a diagram showing an example of arrhythmia diagnosis information.
FIG. 16 is a diagram showing an example of a processing procedure performed by an intracardiac electrocardiogram evaluation apparatus.
FIG. 17 is a diagram showing an example of a generation processing procedure of a first learning model.

### Description of Embodiments

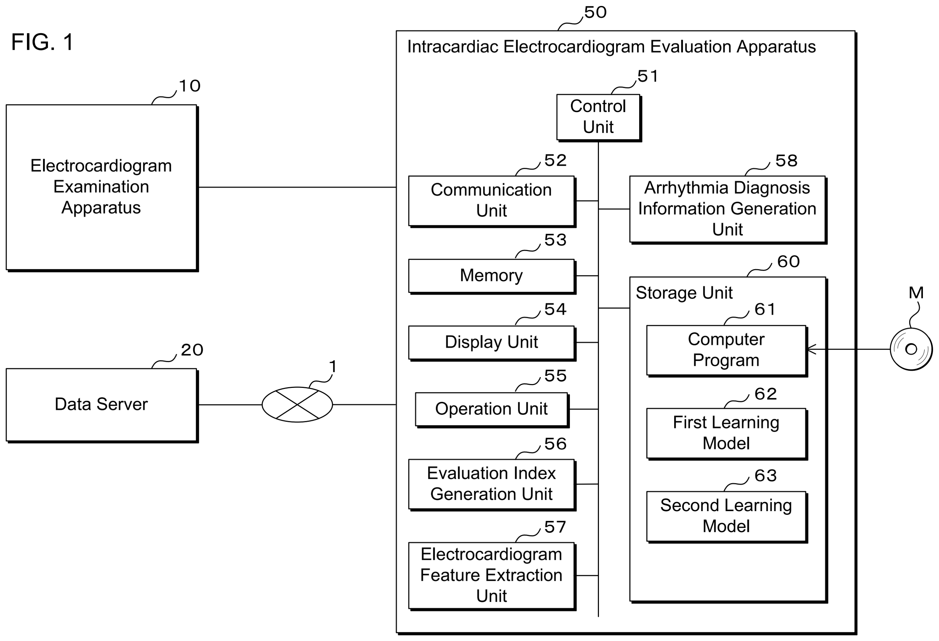

Hereinafter, the present invention will be described with reference to the diagrams showing embodiments thereof. FIG. 1 is a diagram showing an example of the configuration of an intracardiac electrocardiogram evaluation system. The intracardiac electrocardiogram evaluation system includes an intracardiac electrocardiogram evaluation apparatus 50 and an electrocardiogram examination apparatus 10. The intracardiac electrocardiogram evaluation apparatus 50 is connected to the electrocardiogram examination apparatus 10 through wired or wireless communication. In addition, the intracardiac electrocardiogram evaluation apparatus 50 is connected to a data server 20 through a communication network 1.

The electrocardiogram examination apparatus 10 records body surface electrocardiogram data of a subject (such as a patient) and outputs the body surface electrocardiogram data to the intracardiac electrocardiogram evaluation apparatus 50. The electrocardiogram examination apparatus 10 may be a 12-lead electrocardiograph, a Holter electrocardiograph, a portable electrocardiograph, a wearable terminal, and the like. In addition, the electrocardiogram examination apparatus 10 may be made to have the function of the intracardiac electrocardiogram evaluation apparatus 50.

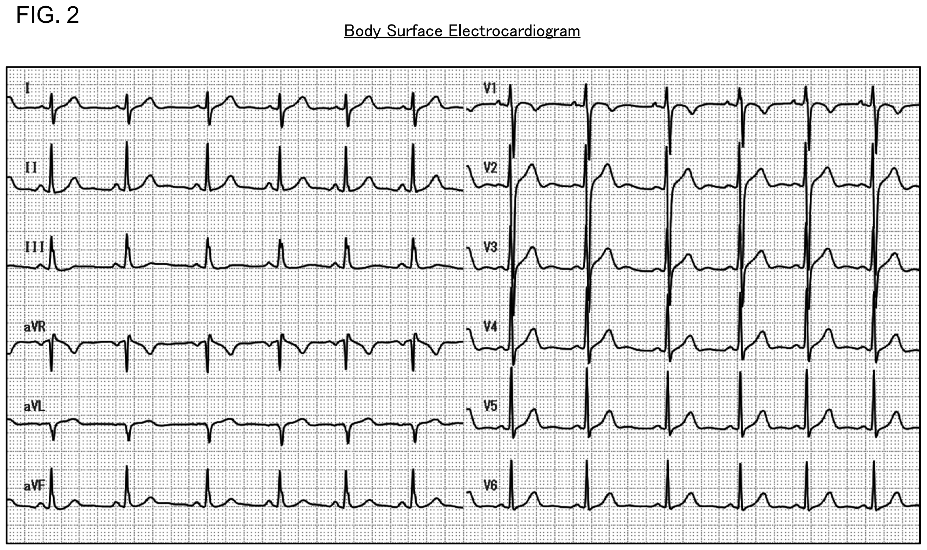

FIG. 2 is a diagram showing an example of body surface electrocardiogram data. FIG. 2 shows 12-lead electrocardiogram data as an example of body surface electrocardiogram data. Electricity generated in the sinoatrial node flows through the atria, atrioventricular node, and ventricles (right and left bundle branches), and the body surface electrocardiogram is time-series data recording the potential differences detected from the body surface for each site. In principle, contraction of the atria or ventricles occurs during depolarization, and expansion occurs during repolarization. The 12-lead electrocardiogram observes the heart through this electrical activity from 12 different directions on the body surface. There are two lead methods for the 12-lead electrocardiogram examination: limb leads and chest leads. For limb leads, electrodes are attached to the left and right wrists and ankles. From the four electrodes, six waveforms "I: leftward, II: left downward, III: right downward, aVR: right upward, aVL: left upward, and aVF: downward" are obtained. Chest leads reflect the electrical direction in the horizontal plane, and by attaching six electrodes at predetermined positions on the chest, six waveforms "V1, V2, V3, V4, V5, and V6" are obtained from the six electrodes.

As shown in FIG. 1, the intracardiac electrocardiogram evaluation apparatus 50 includes a control unit 51 that controls the entire apparatus, a communication unit 52, a memory 53, a display unit 54, an operation unit 55, an evaluation index generation unit 56, an electrocardiogram feature extraction unit 57, an arrhythmia diagnosis information generation unit 58, and a storage unit 60.

The control unit 51 is configured by combining a required number of CPUs (Central Processing Units), MPUs (Micro-Processing Units), GPUs (Graphics Processing Units), GPGPUs (General-purpose computing on graphics processing units), and the like. In addition, the control unit 51 may be configured by combining DSPs (Digital Signal Processors), FPGAs (Field-Programmable Gate Arrays), and the like.

The communication unit 52 includes a communication module, and has a function of communicating with the electrocardiogram examination apparatus 10. In addition, the communication unit 52 has a function of communicating with the data server 20 through the communication network 1.

The memory 53 can be configured using a semiconductor memory such as an SRAM (Static Random Access Memory), a DRAM (Dynamic Random Access Memory), or a flash memory.

The display unit 54 can be configured using a liquid crystal display, an organic EL display, or the like. In addition, instead of the display unit 54, an external display device may be connected to the intracardiac electrocardiogram evaluation apparatus 50.

The operation unit 55 is configured using, for example, a keyboard, a mouse, a touchpad, or a touch panel, and can receive an operation on the information displayed on the display unit 54.

The storage unit 60 can be configured using, for example, a hard disk or a semiconductor memory, and can store a computer program (program product) 61, a first learning model 62, a second learning model 63, and required information. The first learning model 62 and the second learning model 63 include a model before learning, a model in the middle of learning, or a trained model.

The computer program 61 is loaded into the memory 53 and executed by the control unit 51. The computer program 61 may be downloaded from an external device through the communication unit 52 and stored in the storage unit 60. In addition, the computer program 61 recorded on a recording medium (for example, an optically readable disk storage medium such as a CD-ROM) may be read by a recording medium reading unit and stored in the storage unit 60. The computer program 61 can be loaded so as to run on a single computer, or can be loaded so as to run on a plurality of computers interconnected through a communication network by being located at one site or distributed across a plurality of sites.

The evaluation index generation unit 56 and the electrocardiogram feature extraction unit 57 can execute their functions by performing processing using the first learning model 62 by the computer program 61. In addition, the arrhythmia diagnosis information generation unit 58 can execute its functions by performing processing using the second learning model 63 by the computer program 61.

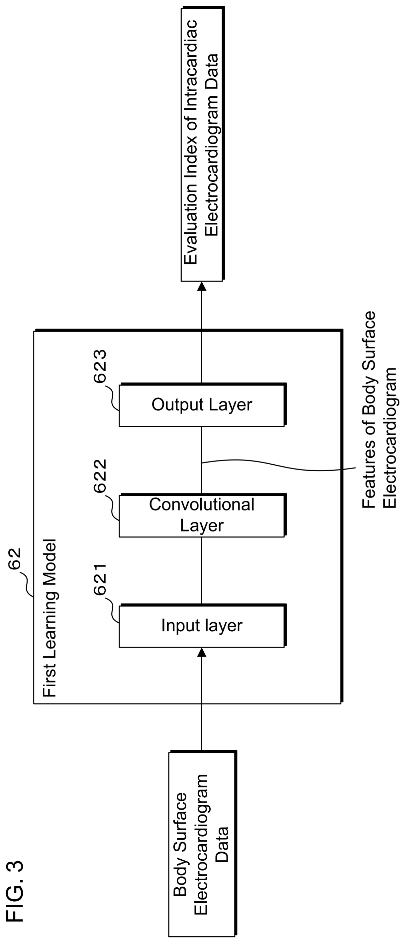

FIG. 3 is a diagram showing an example of the configuration of the first learning model 62. The first learning model 62 includes an input layer 621, a convolutional layer 622, and an output layer 623. The input layer 621 has a predetermined number of channels. When the input body surface electrocardiogram data is 12-lead electrocardiogram data, 12 pieces of time-series data of the 12-lead electrocardiogram data are input to one-dimensional 12 channels of the input layer 621.

The convolutional layer 622 has a plurality of layers, and extracts body surface electrocardiogram features, which are features of the body surface electrocardiogram data, and outputs the extracted body surface electrocardiogram features to the output layer 623. The body surface electrocardiogram features are extracted as features by combining, for example, the shape of each waveform in the body surface electrocardiogram data, the order in which waves (for example, P wave, QRS wave, T wave, and the like) appear, and the intervals between characteristic portions of the waveforms.

The output layer 623 can be configured, for example, as a fully connected layer. By combining the body surface electrocardiogram features output from the convolutional layer 622 into a one-dimensional numerical value, the output layer 623 outputs a probability indicating which label the input body surface electrocardiogram data belongs to. Here, the label is an index (evaluation index) for evaluating the intracardiac electrocardiogram data.

The evaluation index is information obtained by abstracting the intracardiac electrocardiogram data, and is artificially labeled by, for example, an arrhythmia specialist using the intracardiac electrocardiogram data.

The first learning model 62 can be configured using models such as EfficientNet, ResNet, DenseNet, and Vision Transformer, for example. As described above, by performing processing using the first learning model 62, it is possible to execute the function of the evaluation index generation unit 56, which generates an evaluation index of intracardiac electrocardiogram data based on the body surface electrocardiogram data, and the function of the electrocardiogram feature extraction unit 57, which extracts body surface electrocardiogram features, which are features of the body surface electrocardiogram data, based on the body surface electrocardiogram data.

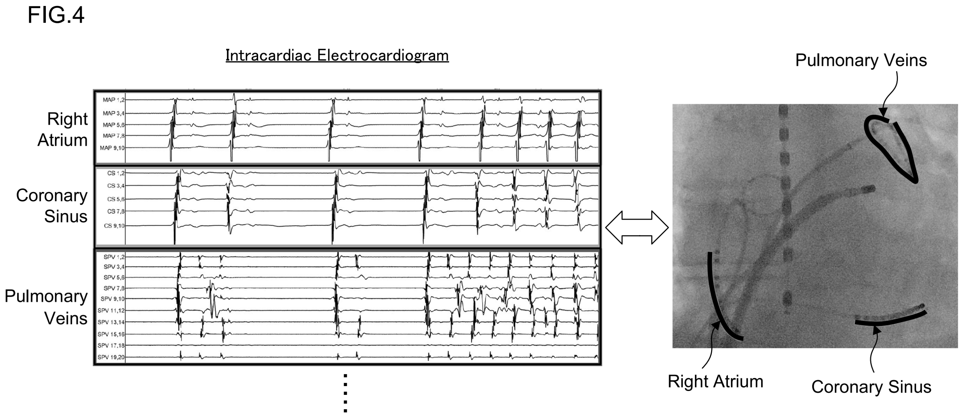

FIG. 4 is a diagram showing an example of intracardiac electrocardiogram data. As sources of intracardiac electrocardiogram data used as evaluation indices of intracardiac electrocardiogram data, for example, there are types such as pulmonary vein potential, coronary sinus potential, right ventricular potential, and ablation catheter potential. Depending on the type of arrhythmia targeted (for example, paroxysmal atrial fibrillation, persistent atrial fibrillation, postoperative recurrent atrial fibrillation, paroxysmal supraventricular tachycardia, ventricular tachycardia, ventricular premature contraction, atrial tachycardia, ventricular fibrillation, or WPS syndrome), different sources of intracardiac electrocardiogram data can be used. The example in FIG. 4 shows intracardiac electrocardiogram data obtained from electrode catheters inserted into predetermined regions of the right atrium (MAP), coronary sinus (CS), and pulmonary veins (SPV). An arrhythmia specialist can abstract and manually classify intracardiac electrocardiogram data, such as those illustrated in FIG. 4, into evaluation indices (labels).

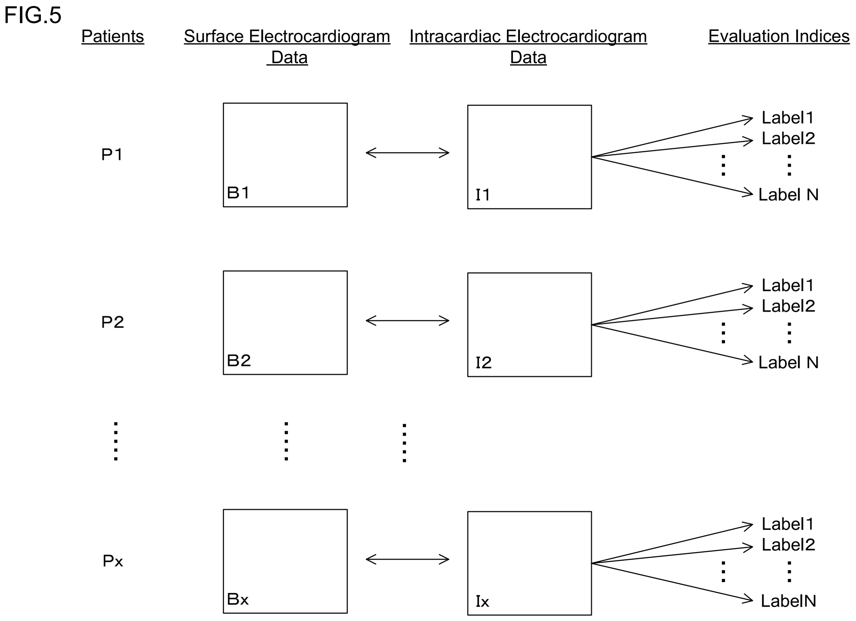

FIG. 5 is a diagram showing an example of creating training data for generating the first learning model 62. Patients (P1, P2, ..., Px) with arrhythmic diseases are registered. Body surface electrocardiogram data (B1, B2, ..., Bx) and intracardiac electrocardiogram data (I1, I2, ..., Ix) of the patients are collected, and the intracardiac electrocardiogram data of each patient is abstracted and labeled. In the example of FIG. 5, the labels are denoted, for convenience, as label 1, label 2, ..., label N. Depending on the patient's arrhythmic disease, the labels for different patients may be different or the same. In this manner, the abstracted intracardiac electrocardiogram data are prepared as correct labels. The training data may be created using an external device (for example, a personal computer). The training data created by the external device can be stored in the data server 20.

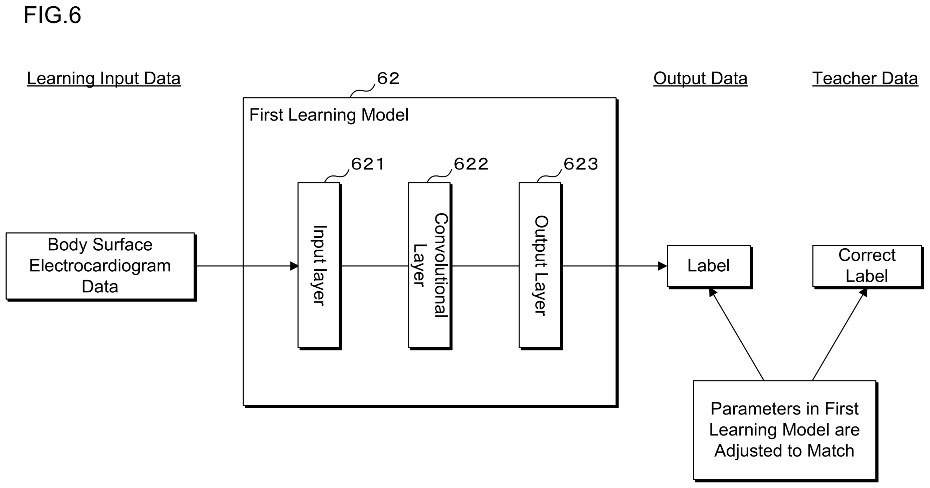

FIG. 6 is a diagram showing an example of a method for generating the first learning model 62. The control unit 51 acquires, from the data server 20, training data including indices (correct labels) for evaluating the body surface electrocardiogram data and the intracardiac electrocardiogram data. Based on the acquired training data, the control unit 51 can generate the first learning model 62 so that, when body surface electrocardiogram data of the subject is input, an index for evaluating intracardiac electrocardiogram data of the subject is output.

Specifically, the body surface electrocardiogram data serving as learning input data is input to the first learning model 62. The parameters in the first learning model 62 are adjusted so that the label (evaluation index) output from the first learning model 62 matches the correct label as teacher data. When the degree of match between the label as output data and the correct label falls within an allowable range, the parameters of the first learning model 62 can be stored in the storage unit 60 to generate the first learning model 62.

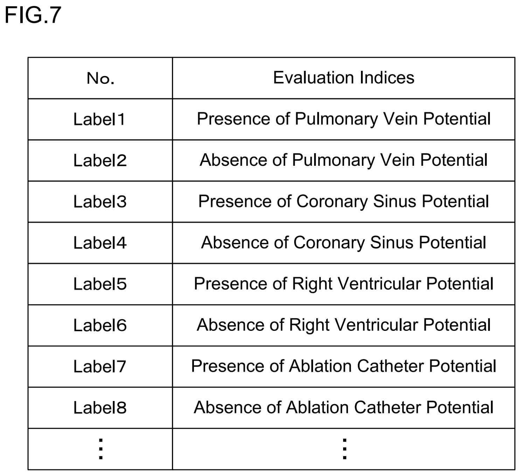

FIG. 7 is a diagram showing a first example of the label. As an example of labels abstracted from intracardiac electrocardiogram data, classification can be made such that, for example, label 1 indicates the presence of pulmonary vein potential, label 2 indicates the absence of pulmonary vein potential, label 3 indicates the presence of coronary sinus potential, label 4 indicates the absence of coronary sinus potential, label 5 indicates the presence of right ventricular potential, label 6 indicates the absence of right ventricular potential, label 7 indicates the presence of ablation catheter potential, label 8 indicates the absence of ablation catheter potential, and so on. In addition, the label numbers are given for convenience and are not limited to the example in FIG. 7.

As described above, the evaluation index (label) includes the presence or absence of a pulmonary vein potential. In addition, the evaluation index may include at least one of the presence or absence of a coronary sinus potential, the presence or absence of a right ventricular potential, and the presence or absence of an ablation catheter potential.

The control unit 51 acquires the body surface electrocardiogram data of the subject, and when the body surface electrocardiogram data is input, the control unit 51 can input the acquired body surface electrocardiogram data to the first learning model 62, which outputs an index (evaluation index, label) for evaluating the intracardiac electrocardiogram data, and output an index for evaluating the intracardiac electrocardiogram data of the subject.

Atrial fibrillation is an arrhythmic disease that occurs when electrical signals are generated disorderly near blood vessels called "pulmonary veins" in the left atrium of the heart. It is thought that, due to aging, cells with abnormal electrical activity appear within the pulmonary veins, and an intense electrical current from these cells flow into the atrium, causing the atrium to lose balance and enter a fibrillatory state.

That is, assuming that the first learning model 62 outputs label 1 when the body surface electrocardiogram data of the subject is input to the first learning model 62, it can be predicted with high accuracy that the subject has an arrhythmia risk caused by atrial fibrillation or a risk of serious events such as cerebral infarction associated therewith. As described above, according to the present embodiment, the presence or absence of an arrhythmic disease or its mechanism can be predicted with high accuracy from the body surface electrocardiogram.

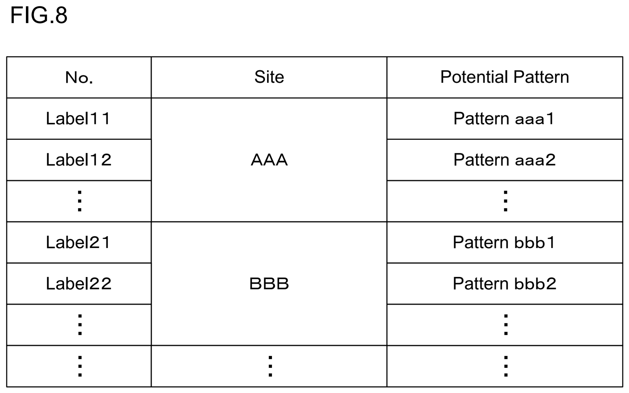

FIG. 8 shows a second example of the label. As an example of labels abstracted from intracardiac electrocardiogram data, for example, label 11 indicates the potential pattern aaa1 at site AAA, label 12 indicates the potential pattern aaa2 at site AAA, ..., label 21 indicates the potential pattern bbb1 at site BBB, label 22 indicates the potential pattern bbb2 at site BBB, .... The label numbers, sites, and potential patterns are given for convenience and are not limited to the example in FIG. 8.

As described above, the evaluation index (label) can include clusters classified by clustering based on potential patterns observed with a plurality of electrodes placed at required sites of the heart. A correct label can be assigned to each cluster classified by clustering.

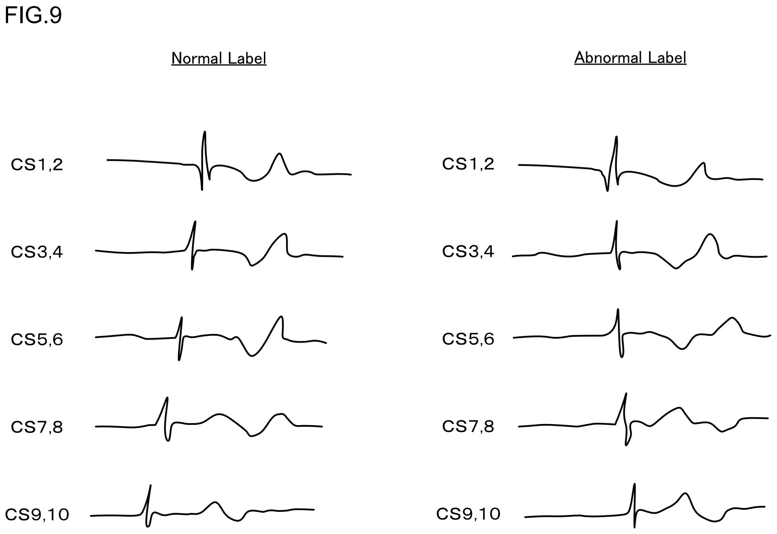

FIG. 9 shows an example of a pattern of coronary sinus potentials. The site AAA is the coronary sinus, the label 11 is the normal label, and the label 12 is the abnormal label. Among the electrodes CS, CS1 and CS2 are on the left side of the heart, and CS9 and CS10 are on the right side of the heart. In the normal label (label 11), the potential pattern flows from CS9 and CS10 to CS1 and CS2 (that is, from the right side of the heart to the left side of the heart) over time. On the other hand, in the abnormal label (label 12), the potential pattern flows from CS1 and CS2 to CS9 and CS10 (that is, from the left side of the heart to the right side of the heart) over time.

That is, assuming that the first learning model 62 outputs label 12 when the body surface electrocardiogram data of the subject is input to the first learning model 62, it can be predicted with high accuracy that the subject has an arrhythmia risk caused by potential imbalance in the coronary sinus or a risk of serious events such as cerebral infarction associated therewith. As described above, according to the present embodiment, the presence or absence of an arrhythmic disease or its mechanism can be predicted with high accuracy from the body surface electrocardiogram.

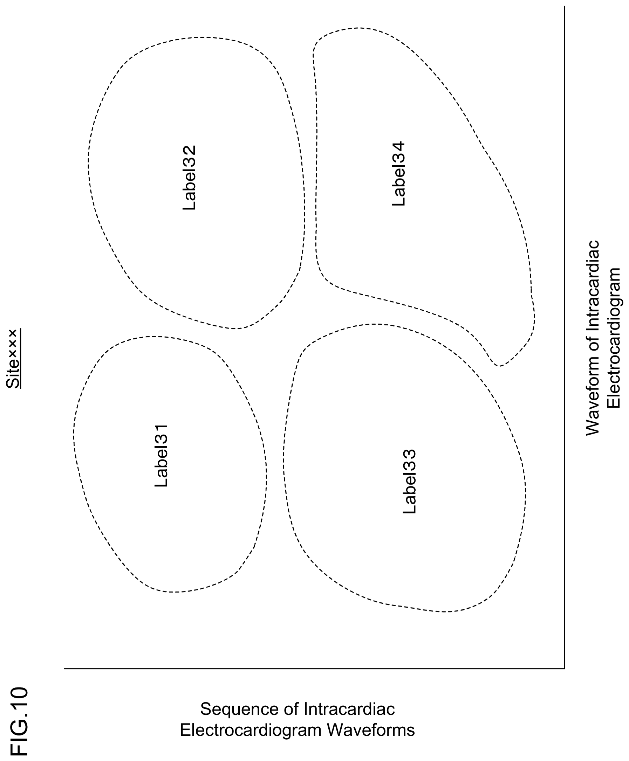

FIG. 10 is a diagram showing a third example of the label. Intracardiac electrocardiogram data is collected from a plurality of patients with arrhythmic diseases, and the intracardiac electrocardiogram data is clustered using the sequence of intracardiac electrocardiogram waveforms at site XXX and the intracardiac electrocardiogram waveform as classification axes. The site XXX may include a plurality of sites. The sequence of intracardiac electrocardiogram waveforms is the temporal order of waveforms at each electrode. The waveform of the intracardiac electrocardiogram includes the shape of the waveform at each electrode, the magnitude of the potential, and the intervals between characteristic points of the waveform. In the example of FIG. 10, for convenience, classification into four clusters with labels 31, 32, 33, and 34 is made. In addition, clustering may be performed using either the sequence of intracardiac electrocardiogram waveforms or the intracardiac electrocardiogram waveforms as a classification axis.

As described above, the evaluation index (label) may include clusters classified by clustering based on at least one of a plurality of intracardiac electrocardiogram waveforms observed with a plurality of electrodes placed at required sites of the heart and the sequence of a plurality of intracardiac electrocardiogram waveforms.

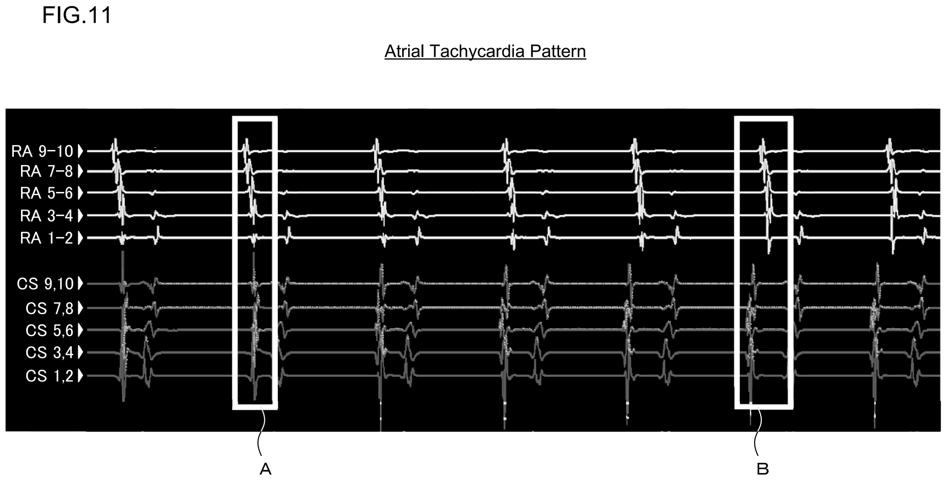

FIG. 11 is a diagram showing an example of an atrial tachycardia pattern. FIG. 11 shows intracardiac electrocardiogram data obtained from electrode catheters inserted into predetermined regions of the right atrium (RA) and coronary sinus (CS). The waveform and the waveform sequence indicated by reference numeral A represent the case of sinus rhythm (normal pattern). The waveform and the waveform sequence indicated by reference numeral B represent an atrial tachycardia pattern. Atrial tachycardia is a type of tachycardia that occurs when myocardial cells with abnormal excitability are present in the atrium and thee myocardial cells excite the heart at a rate exceeding that of the sinoatrial node.

Assuming that the label (evaluation index) of the atrial tachycardia pattern is label 31 and the first learning model 62 outputs label 31 when the body surface electrocardiogram data of the subject is input to the first learning model 62, it can be predicted with high accuracy that the subject has an arrhythmia risk caused by atrial tachycardia or a risk of serious events such as cerebral infarction associated therewith. As described above, according to the present embodiment, the presence or absence of an arrhythmic disease or its mechanism can be predicted with high accuracy from the body surface electrocardiogram.

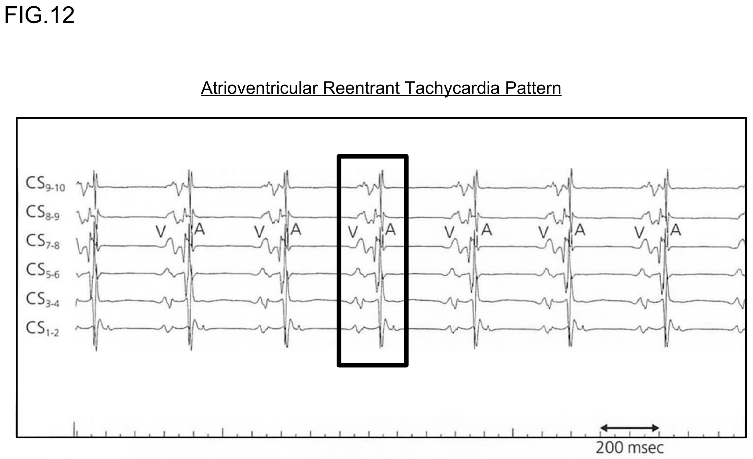

FIG. 12 is a diagram showing an example of an atrioventricular reentrant tachycardia pattern. FIG. 12 shows intracardiac electrocardiogram data obtained from an electrode catheter inserted into a predetermined region of the coronary sinus (CS). The waveform and the waveform sequence within the time period surrounded by the frame indicate the case of an atrioventricular reentrant tachycardia pattern. Atrioventricular reentrant tachycardia (WPS) syndrome) is a type of tachycardia that occurs when the impulse conduction system connecting the atria and ventricles has an accessory pathway in addition to the normal pathway and electrical signals circulate between the normal pathway and the accessory pathway.

Assuming that the label (evaluation index) of the atrioventricular reentrant tachycardia pattern is label 41 and the first learning model 62 outputs label 41 when the body surface electrocardiogram data of the subject is input to the first learning model 62, it can be predicted with high accuracy that the subject has an arrhythmia risk caused by atrioventricular reentrant tachycardia or a risk of serious events such as cerebral infarction associated therewith. As described above, according to the present embodiment, the presence or absence of an arrhythmic disease or its mechanism can be predicted with high accuracy from the body surface electrocardiogram.

Next, visualization of prediction grounds using the first learning model 62 will be described.

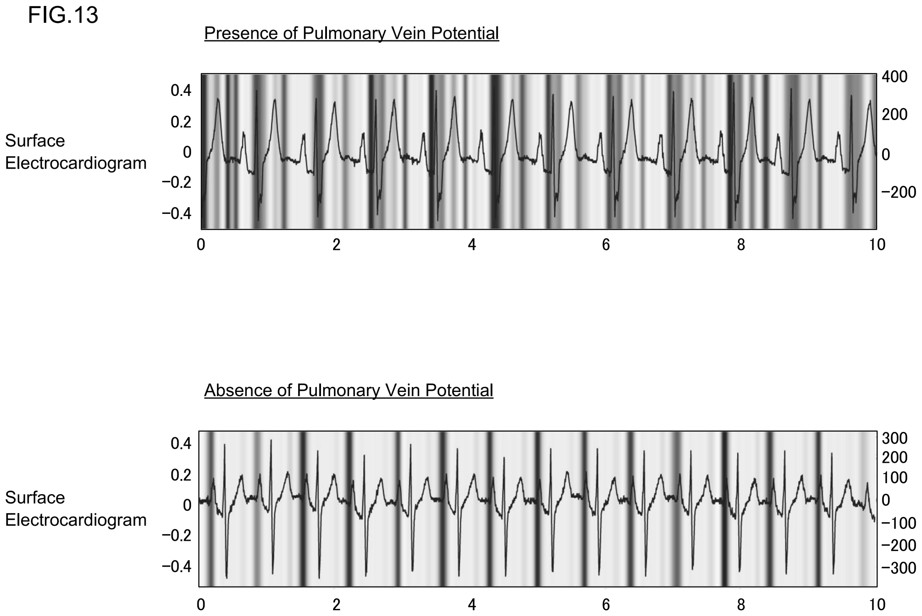

FIG. 13 is a diagram showing an example of visualization of prediction grounds using Grad-CAM. Grad-CAM works by inputting body surface electrocardiogram data to the first learning model 62, extracting a feature map from the first learning model 62, calculating the weight for each channel of the extracted feature map, and summing the weighted channels to generate a saliency map. Since a part in the body surface electrocardiogram data that is more important has higher saliency, it is possible to visualize the prediction grounds.

In FIG. 13, for body surface electrocardiogram data with and without a pulmonary vein potential, the regions determined to be important by the first learning model 62 are shown by different shading. Darker regions indicate higher importance. It can be seen that the prediction grounds are concentrated around time phases close to the appearance location of the pulmonary vein potential. In addition, this is useful for identifying the pulmonary vein potential on the body surface electrocardiogram data.

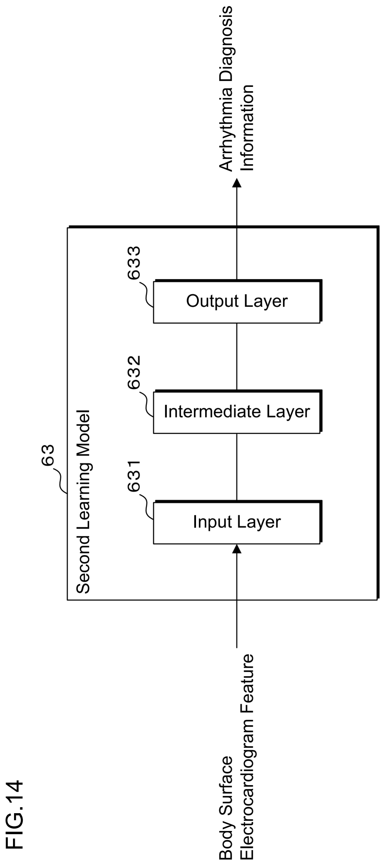

FIG. 14 is a diagram showing an example of the configuration of the second learning model 63. The second learning model 63 includes an input layer 631, an intermediate layer 632, and an output layer 633. The intermediate layer 632 may be a convolutional layer, but does not have to be a convolutional layer. When the body surface electrocardiogram feature extracted by the first learning model 62 is input to the second learning model 63, the second learning model 63 outputs arrhythmia diagnosis information.

That is, the control unit 51 acquires the body surface electrocardiogram feature of the subject, and when the body surface electrocardiogram feature is input, the control unit 51 can input the acquired body surface electrocardiogram feature to the second learning model 63, which outputs arrhythmia diagnosis information, and output arrhythmia diagnosis information of the subject.

By performing processing using the second learning model 63, the function of the arrhythmia diagnosis information generation unit 58 that generates arrhythmia diagnostic information based on the body surface electrocardiogram features is executed.

The second learning model 63 can be generated as follows. That is, the control unit 51 can acquire training data including the body surface electrocardiogram feature and the arrhythmia diagnosis information and generate the second learning model 63 based on the acquired training data so that, when the body surface electrocardiogram feature of the subject is input, the arrhythmia diagnosis information of the subject is output. The training data may be stored in advance in the data server 20.

In addition, although not shown, the second learning model 63 may be generated as follows. That is, the control unit 51 may acquire training data including body surface electrocardiogram data and arrhythmia diagnosis information and generate the second learning model 63 based on the acquired training data so that, when the body surface electrocardiogram data of the subject is input, arrhythmia diagnosis information of the subject is output. The training data may be stored in advance in the data server 20.

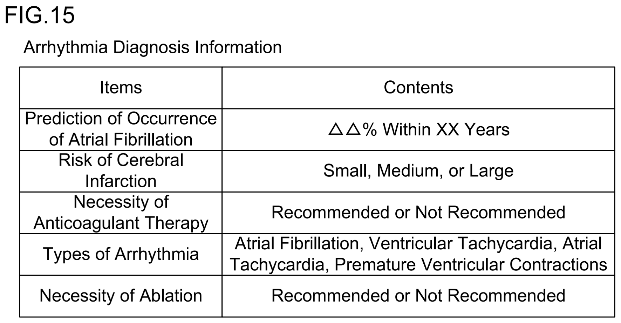

FIG. 15 is a diagram showing an example of arrhythmia diagnosis information. As shown in FIG. 15, the prediction of the occurrence of atrial fibrillation is, for example, △△% within XX years. The risk of cerebral infarction is, for example, small, medium, or large. The necessity of anticoagulant therapy is recommended or not recommended. The types of arrhythmia are atrial fibrillation, ventricular tachycardia, atrial tachycardia, premature ventricular contractions, and the like. The necessity of ablation is recommended or not recommended. In addition, ablation is a treatment for atrial fibrillation using pulmonary vein isolation (PVI). In addition, different second learning models 63 may be used for different items of arrhythmia diagnosis information, or the same second learning model 63 may be used for a plurality of items.

To generate the second learning model 63, medical information such as body surface electrocardiogram data, diagnosis information, treatment information, and intracardiac electrocardiogram data of a plurality of patients with arrhythmic diseases is collected for each patient. By making the second learning model 63 learn, for example, the correlation between the time of acquisition of body surface electrocardiogram data and the onset time of atrial fibrillation based on the medical information of a plurality of patients, it becomes possible to predict the onset of atrial fibrillation in a subject from the body surface electrocardiogram data of the subject.

In addition, by making the second learning model 63 learn, for example, the correlation between the time of acquisition of body surface electrocardiogram data and the presence or absence of cerebral infarction based on the medical information of a plurality of patients, it becomes possible to predict the risk of cerebral infarction of a subject from the body surface electrocardiogram data of the subject.

In addition, by making the second learning model 63 learn, for example, the correlation between body surface electrocardiogram data and treatment information indicating whether or not anticoagulant therapy was required based on the medical information of a plurality of patients, it becomes possible to determine whether or not a subject requires anticoagulant therapy based on the body surface electrocardiogram data of the subject.

In addition, by making the second learning model 63 learn, for example, the correlation between body surface electrocardiogram data and the type of arrhythmia based on intracardiac electrocardiogram data based on the medical information of a plurality of patients, it becomes possible to determine the type of arrhythmia of a subject from the body surface electrocardiogram data of the subject.

In addition, by making the second learning model 63 learn, for example, the correlation between body surface electrocardiogram data and symptoms after ablation based on intracardiac electrocardiogram data based on the medical information of a plurality of patients, it becomes possible to determine whether a subject requires ablation based on the body surface electrocardiogram data of the subject.

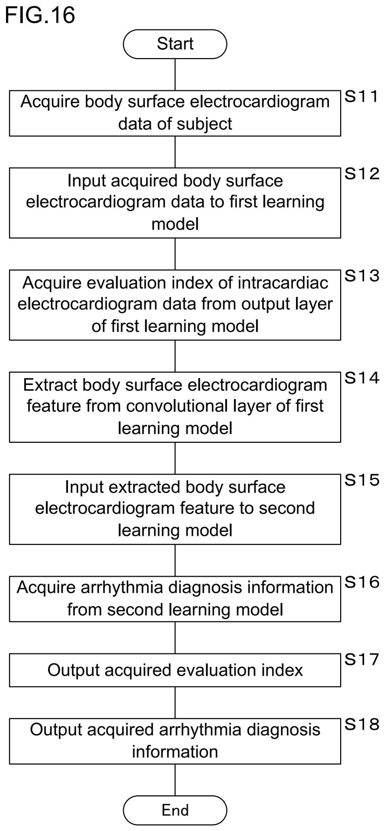

FIG. 16 is a diagram showing an example of a processing procedure performed by the intracardiac electrocardiogram evaluation apparatus 50. For convenience, the processing will be described with the control unit 51 as the main entity. The control unit 51 acquires body surface electrocardiogram data of a subject (S11), and inputs the acquired body surface electrocardiogram data to the first learning model 62 (S12). The control unit 51 acquires an evaluation index of the intracardiac electrocardiogram data from the output layer 623 of the first learning model 62 (S13).

The control unit 51 extracts a body surface electrocardiogram feature from the convolutional layer 622 of the first learning model 62 (S14). The control unit 51 inputs the extracted body surface electrocardiogram feature to the second learning model 63 (S15). The control unit 51 acquires arrhythmia diagnosis information from the second learning model 63 (S16).

The control unit 51 outputs the acquired evaluation index (S17), outputs the acquired arrhythmia diagnosis information (S18), and ends the process. The arrhythmia diagnosis information may be displayed on the display unit 54 or an external display device.

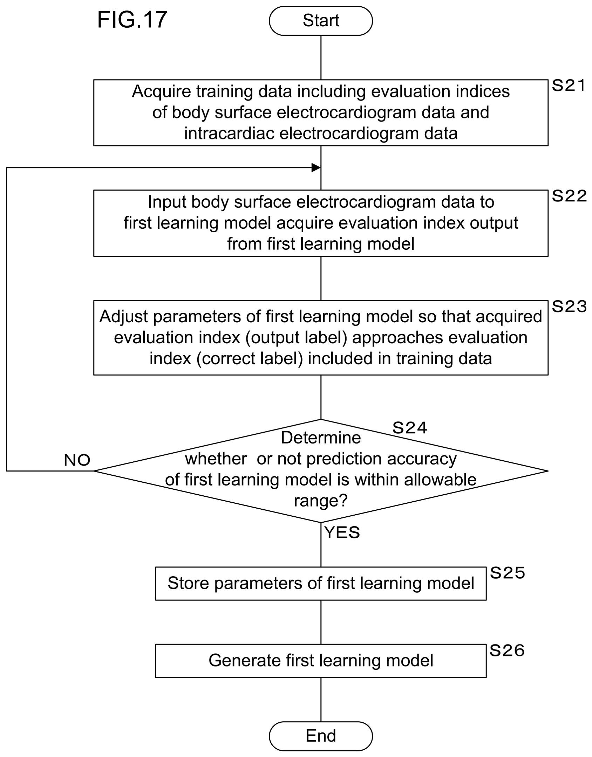

FIG. 17 is a diagram showing an example of a generation processing procedure of the first learning model 62. The control unit 51 acquires training data including evaluation indices (correct labels) of body surface electrocardiogram data and intracardiac electrocardiogram data (S21). The control unit 51 inputs the body surface electrocardiogram data as learning input data to the first learning model 62 and acquires the evaluation index (output label) output from the first learning model 62 (S22).

The control unit 51 adjusts the parameters of the first learning model 62 so that the acquired evaluation index (output label) approaches the evaluation index (correct label) included in the training data (S23). The control unit 51 determines whether or not the prediction accuracy of the first learning model 62 is within an allowable range (S24). If the prediction accuracy is not within the allowable range (NO in S24), the control unit 51 continues the processing from step S22.

If the prediction accuracy is within the allowable range (YES in S24), the control unit 51 stores the parameters of the first learning model 62 in the storage unit 60 (S25), generates the first learning model 62 (S26), and ends the process.

According to the present embodiment, the pulmonary vein potential (a minute potential confirmed by intracardiac electrocardiogram), which is considered to be a cause of atrial fibrillation, can be detected at an appropriate time phase from the body surface electrocardiogram data. This can be used as a basic technology for using clinical arrhythmia prediction models.

(Supplementary Note 1) A computer program causes a computer to execute processing of: acquiring body surface electrocardiogram data of a subject; and inputting the acquired body surface electrocardiogram data to a first learning model, which outputs an index for evaluating intracardiac electrocardiogram data when body surface electrocardiogram data is input, and outputting an index for evaluating intracardiac electrocardiogram data of the subject.

(Supplementary Note 2) In the computer program according to Supplementary Note 1, the index includes presence or absence of a pulmonary vein potential.

(Supplementary Note 3) In the computer program according to Supplementary Note 1 or 2, the index includes at least one of presence or absence of a coronary sinus potential, presence or absence of a right ventricular potential, and presence or absence of an ablation catheter potential.

(Supplementary Note 4) In the computer program according to any one of Supplementary Notes 1 to 3, the index includes clusters classified by clustering based on potential patterns observed with a plurality of electrodes placed at required sites of a heart.

(Supplementary Note 5) In the computer program according to any one of Supplementary Notes 1 to 4, the index includes clusters classified by clustering based on at least one of a plurality of intracardiac electrocardiogram waveforms observed with a plurality of electrodes placed at required sites of a heart and a sequence of the plurality of intracardiac electrocardiogram waveforms.

(Supplementary Note 6) In the computer program according to any one of Supplementary Notes 1 to 5, the computer is caused to execute processing of: extracting an electrocardiogram feature based on the acquired body surface electrocardiogram data; and inputting the extracted electrocardiogram feature to a second learning model, which outputs arrhythmia diagnosis information when an electrocardiogram feature is input, and outputting arrhythmia diagnosis information of the subject.

(Supplementary Note 7) In the computer program according to Supplementary Note 6, the arrhythmia diagnosis information includes at least one of an atrial fibrillation risk, a cerebral infarction risk, and a type of arrhythmia.

(Supplementary Note 8) An intracardiac electrocardiogram evaluation apparatus includes: a control unit; and a first learning model that outputs an index for evaluating intracardiac electrocardiogram data when body surface electrocardiogram data is input, and the control unit acquires body surface electrocardiogram data of a subject, and inputs the acquired body surface electrocardiogram data to the first learning model and outputs an index for evaluating intracardiac electrocardiogram data of the subject.

(Supplementary Note 9) An intracardiac electrocardiogram evaluation system includes: the intracardiac electrocardiogram evaluation apparatus described above; and an electrocardiogram examination apparatus, and the intracardiac electrocardiogram evaluation apparatus acquires body surface electrocardiogram data of the subject from the electrocardiogram examination apparatus.

(Supplementary Note 10) An intracardiac electrocardiogram evaluation method includes: acquiring body surface electrocardiogram data of a subject; and inputting the acquired body surface electrocardiogram data to a first learning model, which outputs an index for evaluating intracardiac electrocardiogram data when body surface electrocardiogram data is input, and outputting an index for evaluating intracardiac electrocardiogram data of the subject.

(Supplementary Note 11) A learning model generation method includes: acquiring training data including indices for evaluating body surface electrocardiogram data and intracardiac electrocardiogram data; and generating a first learning model based on the acquired training data so that, when body surface electrocardiogram data of a subject is input, an index for evaluating intracardiac electrocardiogram data of the subject is output.

The features described in the respective embodiments can be combined with each other. In addition, the independent claims and the dependent claims described in the claims can be combined with each other in all combinations, regardless of the citation form. In addition, although the form of multiple claims that cite two or more other claims (multi-claim form) is used in the scope of claims, the present invention is not limited thereto. A form of multi-claim (multiple claim) that cites at least one multi-claim may also be used.

### Reference Signs List

- 1: communication network
- 10: electrocardiogram examination apparatus
- 20: data server
- 50: intracardiac electrocardiogram evaluation apparatus
- 51: control unit
- 52: communication unit
- 53: memory
- 54: display unit
- 55: operation unit
- 56: evaluation index generation unit
- 57: electrocardiogram feature extraction unit
- 58: arrhythmia diagnosis information generation unit
- 60: storage unit
- 61: computer program
- 62: first learning model
- 63: second learning model

## Claims

1. A computer program causing a computer to execute processing of:
acquiring body surface electrocardiogram data of a subject; and
inputting the acquired body surface electrocardiogram data to a first learning model, which outputs an index for evaluating intracardiac electrocardiogram data when body surface electrocardiogram data is input, and outputting an index for evaluating intracardiac electrocardiogram data of the subject.

2. The computer program according to claim 1,
wherein the index includes presence or absence of a pulmonary vein potential.

3. The computer program according to claim 1,
wherein the index includes at least one of presence or absence of a coronary sinus potential, presence or absence of a right ventricular potential, and presence or absence of an ablation catheter potential.

4. The computer program according to claim 1,
wherein the index includes clusters classified by clustering based on potential patterns observed with a plurality of electrodes placed at required sites of a heart.

5. The computer program according to claim 1,
wherein the index includes clusters classified by clustering based on at least one of a plurality of intracardiac electrocardiogram waveforms observed with a plurality of electrodes placed at required sites of a heart and a sequence of the plurality of intracardiac electrocardiogram waveforms.

6. The computer program according to any one of claims 1 to 5,
wherein the computer is caused to execute processing of:
extracting an electrocardiogram feature based on the acquired body surface electrocardiogram data; and
inputting the extracted electrocardiogram feature to a second learning model, which outputs arrhythmia diagnosis information when an electrocardiogram feature is input, and outputting arrhythmia diagnosis information of the subject.

7. The computer program according to claim 6,
wherein the arrhythmia diagnosis information includes at least one of an atrial fibrillation risk, a cerebral infarction risk, and a type of arrhythmia.

8. An intracardiac electrocardiogram evaluation apparatus, comprising:
a control unit; and
a first learning model that outputs an index for evaluating intracardiac electrocardiogram data when body surface electrocardiogram data is input,
wherein the control unit acquires body surface electrocardiogram data of a subject, and inputs the acquired body surface electrocardiogram data to the first learning model and outputs an index for evaluating intracardiac electrocardiogram data of the subject.

9. An intracardiac electrocardiogram evaluation system, comprising:
the intracardiac electrocardiogram evaluation apparatus according to claim 8; and
an electrocardiogram examination apparatus,
wherein the intracardiac electrocardiogram evaluation apparatus acquires body surface electrocardiogram data of the subject from the electrocardiogram examination apparatus.

10. An intracardiac electrocardiogram evaluation method, comprising:
acquiring body surface electrocardiogram data of a subject; and
inputting the acquired body surface electrocardiogram data to a first learning model, which outputs an index for evaluating intracardiac electrocardiogram data when body surface electrocardiogram data is input, and outputting an index for evaluating intracardiac electrocardiogram data of the subject.

11. A learning model generation method, comprising:
acquiring training data including indices for evaluating body surface electrocardiogram data and intracardiac electrocardiogram data; and
generating a first learning model based on the acquired training data so that, when body surface electrocardiogram data of a subject is input, an index for evaluating intracardiac electrocardiogram data of the subject is output.
